# FASCICULE DE BREVET EUROPEEN

(11) **EP 4 132 357 B1**
(45) Date de publication et mention de la délivrance du brevet: **25.06.2025**
(21) Numéro de dépôt: 21723332.9
(22) Date de dépôt: 06.04.2021
(51) Int. Cl.: A61B 5/145, A61B 5/00, A61M 37/00

(54) **DISPOSITIF DE MESURE D'ANALYTE COMPRENANT UN PATCH ADHESIF**
ANALYTMESSVORRICHTUNG MIT EINEM KLEBEPFLASTER
ANALYTE MEASURING DEVICE COMPRISING AN ADHESIVE PATCH

(30) Priorité: 06.04.2020 FR 2003419
(43) Date de publication de la demande: 15.02.2023
(73) Titulaire: WIZP AS, 3183 Horten (NO)
(72) Inventeur: PIERART, Luc, 94800 Villejuif (FR); LE, Anh-Minh, 92200 Neuilly sur Seine (FR); LY HAK, Kevin, 94350 Villiers sur Marne (FR)
(74) Mandataire: Regimbeau
(86) Numéro de dépôt international: PCT/FR2021/050598
(87) Numéro de publication internationale: WO 2021/205110

(56) Documents cités:
- EP-A1- 3 603 512
- WO-A1-2019/121324
- US-A1- 2015 190 074

## Description

### DOMAINE DE L'INVENTION

La présente invention se rapporte un système de surveillance corporelle via analyse de liquide corporel, typiquement interstitiel, à l'aide de microaiguilles. Plus précisément, la présente invention concerne un patch pour la gestion du maintien des microaiguilles dans la peau.

### ETAT DE LA TECHNIQUE

Certaines pathologies comme le diabète nécessitent une surveillance quotidienne de paramètres biochimiques du corps humain, *i.e.* des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Ce dispositif portatif, typiquement au poignet, permet une mesure en continu et il suffit de changer la capsule pour changer de microaiguilles.

Toutefois, lorsqu'un tel dispositif est porté au poignet, une difficulté réside dans l'ajustement préalable du dispositif à un endroit confortable pour l'utilisateur. En effet, l'ajustement du dispositif est difficilement compatible avec la présence de microaiguilles dans la peau car les microaiguilles peuvent venir endommager la peau lors de leur insertion.

Des dispositifs de l'art antérieur comprennent un patch, mais pas de moyen d'ajustement sur un membre de l'utilisateur. De tels dispositifs sont également souvent appliqués sur la peau en une seule prise avec un applicateur, sans possibilité de réajustement. Le document WO2019121324 décrit un autre dispositif de l'art antérieur.

### EXPOSE DE L'INVENTION

Un but de l'invention est de proposer une solution pour fabriquer système de surveillance corporelle permettant de résoudre au moins en partie les problèmes de l'art antérieur précités.

Ce but est atteint dans le cadre de la présente invention grâce à un système de surveillance corporelle, comprenant :
- une capsule de mesure d'un analyte corporel, la capsule comprenant une première face propre à être mise en contact avec la peau d'un utilisateur, et des microaiguilles montées fixes sur la première face,
- un patch comprenant un corps principal, le corps principal comprenant une deuxième face propre à être en regard de la peau d'un utilisateur, le patch comprenant une couche d'adhésif recouvrant au moins en partie la deuxième face, et un pelable recouvrant la couche d'adhésif,
- le pelable comprenant au moins deux parties disjointes, les parties étant adjacentes entre-elles,
- le corps principal et la couche d'adhésif comprenant une partie centrale dans laquelle est formé un ajour propre à recevoir la capsule, l'ajour présentant un diamètre maximal supérieur à 2 mm, préférentiellement supérieur à 5 mm,
- le pelable recouvrant entièrement l'ajour,
- la capsule étant reçue de manière amovible dans l'ajour, et
- le pelable recouvrant les microaiguilles.

L'invention est avantageusement complétée par les caractéristiques suivantes, prises individuellement ou en l'une quelconque de leurs combinaisons techniquement possibles :
- le patch s'étend principalement selon une direction longitudinale, et les deux parties sont agencées de part et d'autre d'un axe parallèle à la direction longitudinale,
- le patch présente une forme oblongue selon la direction longitudinale,
- chaque partie du pelable comprend une languette adaptée à la préhension par un utilisateur,
- au moins une des languettes est liée à une extrémité du pelable par rapport à la direction longitudinale,
- la languette est montée fixe à une extrémité du pelable,
- au moins une languette s'étend principalement selon un axe principal présentant une composante non nulle selon la direction longitudinale,
- la valeur absolue d'un angle formé entre la direction longitudinale et l'axe principal est préférentiellement comprise entre 30° et 60°,
- le corps principal présente une épaisseur plus élevée qu'une épaisseur de la couche d'adhésif, et préférentiellement une épaisseur supérieure à 50 µm, notamment supérieure à 100 µm,
- le patch présente un contour, et des parties du contour agencées de part et d'autre de l'ajour selon une direction transverse à la direction longitudinale forment des dents de scie,
- le pelable comprend au moins une couche extérieure disposée à l'opposé du corps principal par rapport au reste du pelable, la couche extérieure recouvre les microaiguilles, le pelable comprenant également au moins un espaceur agencé au moins en partie autour de l'ajour, une épaisseur de l'espaceur étant configurée pour surélever une partie de la couche extérieure recouvrant l'ajour par rapport au reste de la couche extérieure de sorte à former un espace entre la couche extérieure et un sommet de chaque microaiguille,
- le système comprend deux espaceurs agencés de part et d'autre de l'ajour selon une direction transverse à la direction longitudinale, chaque espaceur s'étendant principalement selon la direction longitudinale.

Un autre aspect de l'invention est un dispositif destiné à être attaché à un membre, comprenant :
- un système conforme à l'invention,
- un boitier apte à être fixé de manière amovible avec la capsule du système, à l'intérieur duquel se trouve une batterie et un processeur, le processeur étant configuré pour traiter des données obtenues à l'aide de la mesure faite par au moins une des microaiguilles,
- une lanière, configurée pour maintenir le boitier en place sur le membre.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
La figure 1 illustre schématiquement un patch selon un mode de réalisation de l'invention, en vue de côté.
La figure 2 illustre schématiquement un patch selon un mode de réalisation de l'invention, en vue de dessous.
La figure 3 illustre schématiquement un système de surveillance corporelle comprenant une capsule et un patch selon un mode de réalisation de l'invention, en coupe.
La figure 4 illustre schématiquement un patch comprenant des languettes, selon un mode de réalisation de l'invention, en vue de dessous.
La figure 5 illustre schématiquement un patch présentant des contours en dents de scie, selon un mode de réalisation de l'invention, en vue de dessous.
La figure 6 illustre schématiquement un système de surveillance corporelle selon un mode de réalisation de l'invention, en coupe, dans lequel le patch comprend un espaceur.
La figure 7 illustre schématiquement différentes couches, en vue en éclatée, formant un patch selon un mode de réalisation de l'invention.
La figure 8 illustre schématiquement différentes couches, en vue en éclatée, formant un patch selon un mode de réalisation de l'invention.

Sur l'ensemble des figures, les éléments similaires portent des références identiques.

### DEFINITIONS

On entend par « diamètre » d'un ajour la taille maximale de cet ajour, quelle que soit la forme de l'ajour, soit la distance maximale entre deux points d'un contour de l'ajour.

### DESCRIPTION DETAILLEE DE L'INVENTION

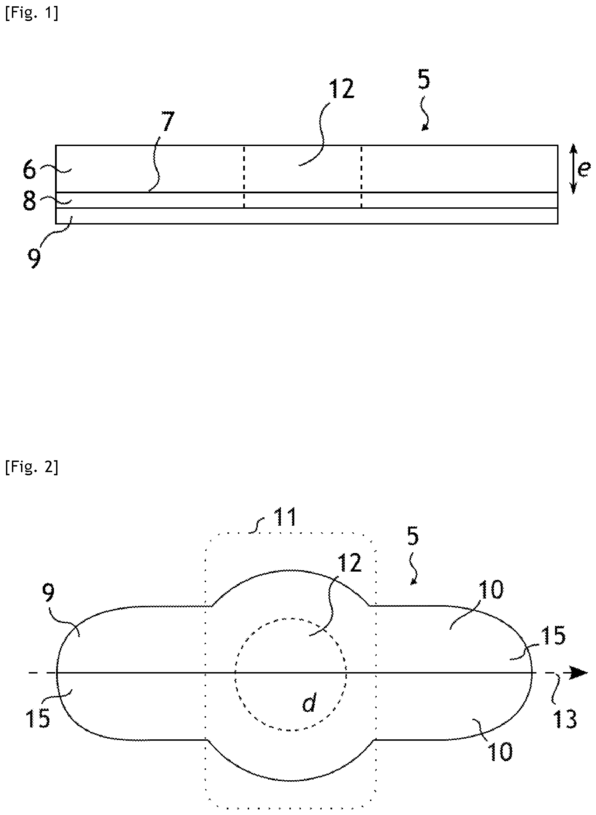

En référence à la figure 1 et à la figure 2, un patch 5 comprend un corps principal 6. Le corps principal 6 est de forme globale plane, et présente une épaisseur *e*. Le corps principal 6 comprend une deuxième face 7 propre à être en regard de la peau d'un utilisateur lorsque le système 1 de surveillance corporelle est porté à un membre de l'utilisateur. Le corps principal 5 peut être fabriqué en polyuréthane (PU). Le patch 5 comprend également une première couche d'adhésif 8 recouvrant au moins en partie, préférentiellement totalement, la deuxième face 7 du corps principal 6.

La première couche d'adhésif 8 comprend préférentiellement un adhésif permettant de réutiliser le patch 5 sur la peau de l'utilisateur. L'adhésif de la première couche d'adhésif 8 peut comprendre préférentiellement de la silicone.

La patch 5 comprend également un pelable 9 recouvrant la première couche d'adhésif 8. On entend par « pelable » un revêtement de surface qui peut être détaché par voie sèche et sans rupture. En d'autres termes, le pelable 9 est destiné à être détaché de la première couche d'adhésif 8 par l'utilisateur, et permet de protéger celle-ci lorsqu'il la recouvre.

Le corps principal 6 et la première couche d'adhésif 8 comprennent une partie centrale 11. Un ajour 12 est formé dans la partie central 11. L'ajour 12 est destiné à recevoir une capsule 2.

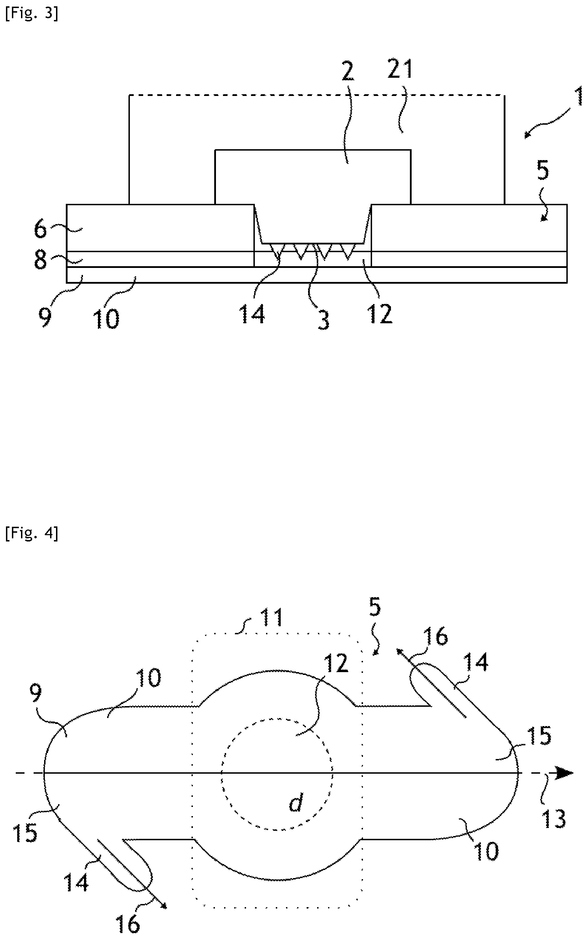

En référence à la figure 3, le système 1 de détection corporelle comprend le patch 5 et une capsule 2 de mesure d'analyte corporel. La capsule 2 comprend une première face 3, propre à être mise en contact avec la peau d'un utilisateur. Des microaiguilles 4 sont montées fixes sur la première face 3. Par « microaiguille », on entend une aiguille présentant une base et un sommet, et dont la base présente une taille inférieure à 600 µm, et dont la distance de la base au sommet de l'aiguille est inférieure à 1 mm, préférentiellement inférieure à 800 µm. La première face 3 n'est pas limitée à une face plane. Elle peut présenter un ou plusieurs renfoncements, dans lequel ou lesquels la base de chaque microaiguille 4 peut être montée fixe.

L'ajour 12 du patch 5 est configuré pour recevoir de manière amovible la capsule 2, comme illustré dans la figure 3. L'ajour 12 présente un diamètre maximal supérieur à 2 mm, notamment supérieur à 5 mm, et préférentiellement supérieur à 1 cm. Ainsi, la capsule 2 peut être logée dans l'ajour 12.

En référence à la figure 2, le pelable 9 comprend au moins deux parties 10 disjointes, chacune des parties étant adjacentes entre-elles. Le pelable 9 recouvre entièrement à la fois l'ajour 12, et les microaiguilles 4 de la capsule 2.

Cette configuration du système 1 de surveillance corporelle permet à la fois de le retrait du pelable 9 après avoir disposé le système 1 en regard de la peau de l'utilisateur, sans endommager les microaiguilles 4, de par la séparation du pelable 9 en plusieurs parties disjointes, et à la fois de protéger les microaiguilles 4 de l'environnement extérieur de par la couverture des microaiguilles 4 par le pelable 9.

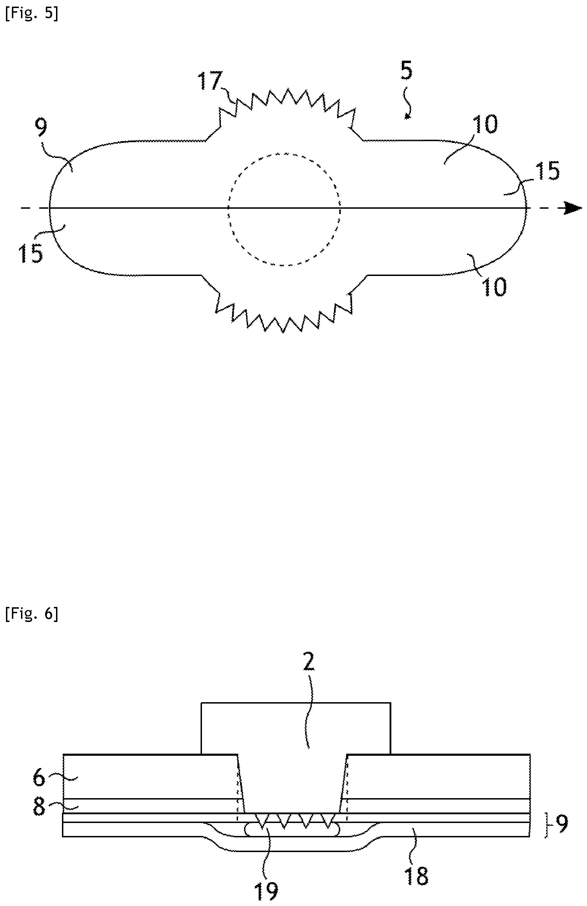

En référence à la figure 2, à la figure 4 et à la figure 5, le patch 5 est préférentiellement allongé selon une direction longitudinale 13, ou en d'autres termes s'étend principalement selon une direction longitudinale 13. Par « s'étend principalement selon une direction », on entend que la taille maximale du patch est décrite par ladite direction. Ainsi, il est possible d'augmenter la partie du patch 5 présentant une première couche d'adhésif 8 et d'augmenter l'adhérence du système 1 sur la peau pour une taille de capsule prédéterminée. Le patch 5 présente préférentiellement une forme oblongue selon la direction longitudinale 13. Ainsi, le patch 5 ne forme pas d'angle susceptible de se décoller de la peau de l'utilisateur.

En référence à la figure 2, le patch 5 comprend préférentiellement deux parties 10, chaque partie 10 étant agencée de part et d'autre d'un axe parallèle à la direction longitudinale 13. Ainsi, une fois le système disposé en regard de la peau par l'utilisateur, il est possible de décoller chacune des parties 10 du reste du patch 5 plus facilement et sans endommager les microaiguilles 4 de la capsule 2.

En variante, le patch 5 peut également comprendre quatre parties, chaque partie étant adjacentes à deux autres parties, les parties 10 se rejoignant au centre du patch 5. Les quatre parties 10 forment alors des quarts de la surface du patch 5. Ainsi, le retrait du pelable 9 lorsque le système est disposé par l'utilisateur en regard de la peau est facilité.

En référence à la figure 4, au moins une partie 10 du pelable 9, et préférentiellement chaque partie 10 du pelable 9 comprend une languette 14 adaptée à la préhension par un utilisateur. Cette languette se démarque préférentiellement de la forme du reste du patch 5. Ainsi, lorsque le système 1 est mis en regard de la peau de l'utilisateur et ajusté à un endroit propice pour une mesure, l'utilisateur peut tirer sur la languette 14 de manière à ôter le pelable du reste du patch 5 sans avoir à déplacer le patch 5 pour le décoller.

La languette 14 peut être fabriquée par les méthodes suivantes, de manière non-limitative. La forme de découpe du pelable 9 peut être réalisée de manière à former la languette 14 dans le même plan et le même matériau qu'une partie du reste du pelable, de manière monobloc. En variante, la languette 14 peut être collée ou fixée à une partie du pelable. L'agencement de la languette 14 peut être contrôlé par un pliage de la languette.

Lorsque le patch 5 présente une forme allongée, il présente deux extrémités 15. La languette est préférentiellement liée, ou montée fixe, à l'extrémité 15 du patch 5. Ainsi, il est possible d'ôter le pelable 9 du reste du patch 5 en tirant sur la languette 14 et en la ramenant vers le centre du système 1.

La languette 14 s'étend préférentiellement selon un axe principal 16, l'axe principale présentant une composante non-nulle selon la direction longitudinale 13. Ainsi, lors de la traction de la languette 14 par un utilisateur, le décollement du pelable à son extrémité 15 est favorisé, et le décollement du pelable 14 du reste du patch 5 est ainsi facilité. La valeur absolue d'un angle formé entre la direction longitudinale 13 et l'axe principal 16 est préférentiellement comprise entre 30° et 60°.

En référence à la figure 1, le corps principal 6 présente une épaisseur e plus élevée qu'une épaisseur de la première couche d'adhésif 8, et préférentiellement une épaisseur supérieure à 50 µm, notamment supérieure à 100 µm. L'épaisseur *e* du corps principal 6 est notamment plus grande que l'épaisseur de n'importe quelle autre couche formant le patch 5. Ainsi, la rigidité du patch 5 est assurée lors de retrait du pelable par l'utilisateur.

En référence à la figure 5, le patch 5 présente un contour 17. Le patch 5 peut comprendre des parties du contour 17, agencées de part et d'autre de l'ajour 12 selon une direction transverse à la direction longitudinale 13, formant des dents de scie. Les inventeurs ont en effet découvert en comparant deux patchs 5 présentant le même pouvoir adhésif à la peau d'un utilisateur, qu'un patch 5 présentant des parties de son contour en dents de scie entraîne une douleur moindre lors du décollage du patch de la peau de l'utilisateur qu'un patch ne présentant pas ce type de contour. En effet, les dents de scies permettent de diminuer le contact de surface avec la peau et donc l'arrachage des poils du l'utilisateur. Par « dents de scie », on entend que le contour 17 du patch 5 présente une forme crantée. Préférentiellement, les crans de la forme crantée présentent une longueur maximale de 5 mm. Chaque cran peut présenter un sommet arrondi. L'angle formé par deux crans successifs est préférentiellement compris entre 20° et 70°.

En référence à la figure 6, le pelable 9 comprend préférentiellement au moins une couche extérieure 18 disposée à l'opposé du corps principal 6 par rapport au reste du pelable 9. La couche extérieure 18 recouvre au moins l'ajour 12 et les microaiguilles 4. Le pelable 9 comprend également au moins un espaceur 19 agencé au moins en partie autour de l'ajour 12. L'espaceur 19 présente une épaisseur configurée pour surélever une partie de la couche extérieure 18 recouvrant l'ajour 12 par rapport au reste de la couche extérieure 18, de sorte à former un espace entre la couche extérieure 18 et le sommet de chaque microaiguille 4. Cette configuration permet de recouvrir les microaiguilles 4 de manière à les protéger avant l'utilisation du système, tout en permettant d'aligner la première couche d'adhésif 8 avec la première face 3. En effet, lors de l'utilisation du système 1, ces deux faces sont au contact de la peau et sont préférentiellement alignées dans le système. Toutefois, en l'absence d'espaceur 19, la hauteur des microaiguilles 4 pourrait dépasser d'une surface de la première couche d'adhésif 8 et se trouver en contact du pelable 9, ce qui risquerait d'endommager les microaiguilles 4. L'espace imposé par l'espaceur 19 entre la couche extérieure 18 et le sommet de chaque microaiguille 4 est préférentiellement supérieur à 10 µm, notamment à 100 µm, et plus préférentiellement à 500 µm.

Les différents éléments du pelable 9 peuvent eux-mêmes être divisés en plusieurs parties, de sorte à former les différentes parties 10 du pelable 9. Par exemple, la couche extérieure 18 est préférentiellement divisée en deux ou plus parties adjacentes entre-elles. Ainsi, le système 1 comprend préférentiellement plusieurs espaceurs 19, notamment deux espaceurs 19, agencés de part et d'autre de l'ajour 12. Dans cette configuration, chaque partie 10 du pelable 9 comprend un espaceur 19. Ainsi, lors du retrait du pelable 9 lors de l'utilisation du système 1, l'espaceur 19 ne risque pas de venir endommager les microaiguilles 4.

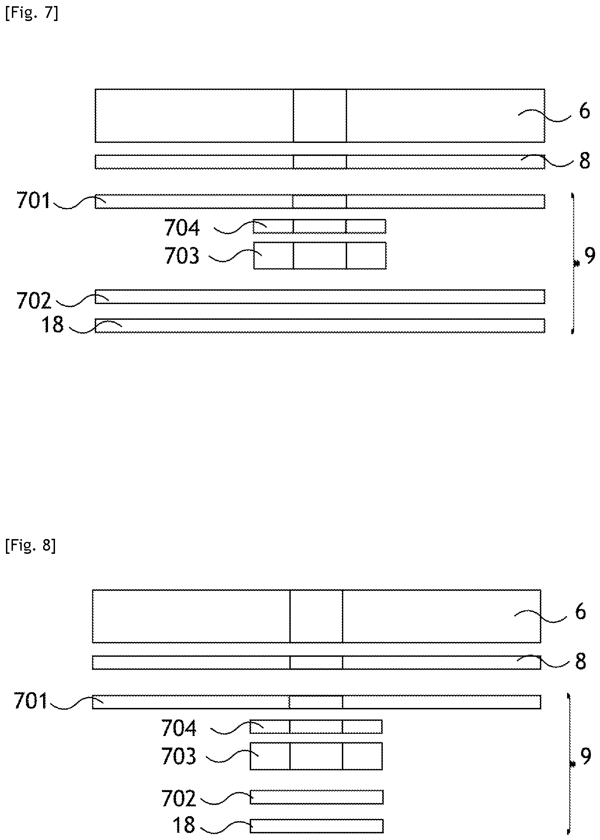

En référence à la figure 7, le pelable 9 comprend préférentiellement, en regard de la première couche d'adhésif 8, un premier revêtement 701, destiné à être en contact avec la première couche d'adhésif 8. Le premier revêtement 701 peut être fabriqué en polyuréthane, en polyéthylène ou tout matériau connu de l'art antérieur.

Le premier revêtement 701 est recouvert, du côté opposé à celui de la première couche d'adhésif 8, par une troisième couche d'adhésif 704. La troisième couche d'adhésif 704 permet de monter fixe un corps d'espaceur 703 sur le premier revêtement 701.

Le corps d'espaceur 703 recouvre la troisième couche d'adhésif 704 du côté opposé au premier revêtement 701. L'espaceur 19 comprend préférentiellement le corps d'espaceur 703 et la troisième couche d'adhésif 704. Le corps d'espaceur 703 est préférentiellement fabriqué en coton.

Une deuxième couche d'adhésif 702 recouvre le corps d'espaceur 703 du côté opposé à la troisième couche d'adhésif 704.

Une deuxième couche d'adhésif 702 peut être agencée entre le corps d'espaceur 703 et la couche extérieure 18. La deuxième couche d'adhésif 702 comprend préférentiellement un adhésif dit fort, tel que de l'acrylique. La deuxième couche d'adhésif 702 permet d'attacher la couche extérieure 18 au premier revêtement 701 et préférentiellement à la fois de maintenir l'espaceur 19 entre la couche extérieure 18 et le premier revêtement 701.

En référence à la figure 8, la deuxième couche d'adhésif 702 et la couche extérieure 18 recouvrir seulement le ou les espaceurs 19, sans être en contact avec le premier revêtement 701.

Un autre aspect de l'invention est un dispositif destiné à être attaché à un membre comprenant un système selon un mode de réalisation de l'invention, un boitier 21 apte à être fixé de manière amovible avec la capsule 2, à l'intérieur duquel se trouve une batterie et un processeur. Le processeur est configuré pour traiter des données obtenues à l'aide de la mesure faite par au moins une des microaiguilles 4. Le dispositif comprend également une lanière configurée pour maintenir le boitier 21 en place sur le membre. Ainsi, il est possible à l'utilisateur, dans un premier temps, placer le système 1 au-dessus de sa peau, de sorte que les microaiguilles 4 soient placées au-dessus d'une partie de la peau propice à rentrer en contact avec les microaiguilles 4, puis ajuster la position du système 1, puis retirer le pelable 9 du reste du système 1, puis finir de serrer la lanière pour fixer le système sur le membre de l'utilisateur.

De manière générale, le système permet ainsi de protéger les microaiguilles 4 d'une contamination éventuelle avec l'environnement avant le contact des microaiguilles 4 avec la peau de l'utilisateur, et de réajuster le dispositif sur un membre de l'utilisateur dans une position plus confortable, sans craindre d'endommager ni la peau ni les microaiguilles.

Le système 1 est préférentiellement adapté pour mesurer une concentration d'un analyte corporel choisi parmi le glucose et le lactate dans le liquide interstitiel d'un utilisateur, et préférentiellement en continu.

## Revendications

1. Système (1) de surveillance corporelle, comprenant :
- une capsule (2) de mesure d'un analyte corporel, la capsule (2) comprenant une première face (3) propre à être mise en contact avec la peau d'un utilisateur, et des microaiguilles (4) montées fixes sur la première face (3),
- un patch (5) comprenant un corps principal (6), le corps principal (6) comprenant une deuxième face (7) propre à être en regard de la peau d'un utilisateur, le patch (5) comprenant une première couche d'adhésif (8) recouvrant au moins en partie la deuxième face (7), et un pelable (9) recouvrant la couche d'adhésif (8),
**caractérisé en ce que** :
- le pelable (9) comprend au moins deux parties (10) disjointes, les parties (10) étant adjacentes entre-elles,
- le corps principal (6) et la première couche d'adhésif (8) comprennent une partie centrale (11) dans laquelle est formé un ajour (12) propre à recevoir la capsule (2), l'ajour (12) présentant un diamètre maximal supérieur à 2 mm, préférentiellement supérieur à 5 mm,
- le pelable (9) recouvre entièrement l'ajour (12),
- la capsule (2) est reçue de manière amovible dans l'ajour (12), et
- le pelable (9) recouvre les microaiguilles (4).

2. Système (1) selon la revendication 1, dans lequel le patch (5) s'étend principalement selon une direction longitudinale (13), et dans lequel les deux parties (10) sont agencées de part et d'autre d'un axe parallèle à la direction longitudinale (13).

3. Système (1) selon l'une des revendications 1 ou 2, dans lequel le patch présente une forme oblongue selon la direction longitudinale (13).

4. Système (1) selon l'une des revendications 1 à 3, dans lequel chaque partie (10) du pelable (9) comprend une languette (14) adaptée à la préhension par un utilisateur.

5. Système (1) selon la revendication 4, dans lequel au moins une des languettes (14) est liée à une extrémité (15) du pelable (9) par rapport à la direction longitudinale (13).

6. Système (1) selon l'une des revendications 4 ou 5, dans lequel la languette (14) est montée fixe à une extrémité (15) du pelable (9).

7. Système (1) selon la revendication 5 ou 6, dans lequel au moins une languette (14) s'étend principalement selon un axe principal (16) présentant une composante non nulle selon la direction longitudinale (13).

8. Système (1) selon la revendication 7, dans lequel la valeur absolue d'un angle formé entre la direction longitudinale (13) et l'axe principal (16) est préférentiellement comprise entre 30° et 60°.

9. Système (1) selon l'une des revendications 1 à 8, dans lequel le corps principal (6) présente une épaisseur plus élevée qu'une épaisseur de la couche d'adhésif (8), et préférentiellement une épaisseur supérieure à 50 µm, notamment supérieure à 100 µm.

10. Système (1) selon l'une des revendications 1 à 9, dans lequel le patch (5) présente un contour (17), et dans lequel des parties du contour (17) agencées de part et d'autre de l'ajour (12) selon une direction transverse à la direction longitudinale (13) forment des dents de scie.

11. Système (1) selon l'une des revendications 1 à 10, dans lequel le pelable (9) comprend au moins une couche extérieure (18) disposée à l'opposé du corps principal (6) par rapport au reste du pelable (9), la couche extérieure (18) recouvrant les microaiguilles (4), le pelable comprenant également au moins un espaceur (19) agencé au moins en partie autour de l'ajour (12), une épaisseur de l'espaceur étant configurée pour surélever une partie de la couche extérieure (18) recouvrant l'ajour (12) par rapport au reste de la couche extérieure (18) de sorte à former un espace entre la couche extérieure (18) et un sommet de chaque microaiguille (4).

12. Système (1) selon la revendication 11, comprenant deux espaceurs (19) agencés de part et d'autre de l'ajour (12) selon une direction transverse à la direction longitudinale (13), chaque espaceur s'étendant principalement selon la direction longitudinale (13).

13. Dispositif (20) destiné à être attaché à un membre, comprenant :
- un système (1) conforme à l'une des revendications 1 à 12,
- un boitier (21), apte à être fixé de manière amovible avec la capsule (2), à l'intérieur duquel se trouve une batterie et un processeur, le processeur étant configuré pour traiter des données obtenues à l'aide de la mesure faite par au moins une des microaiguilles (4),
- une lanière (22) configurée pour maintenir le boitier (21) en place sur le membre.

## Patentansprüche

1. Körperüberwachungssystem (1), umfassend:
- eine Kapsel (2) zum Messen eines Körperanalyts, wobei die Kapsel (2) eine erste Seite (3) umfasst, die imstande ist, mit der Haut eines Benutzers in Kontakt versetzt zu werden, und Mikronadeln (4) die fest auf der ersten Seite (3) angebracht sind,
- ein Pflaster (5), das einen Hauptkörper (6) umfasst, wobei der Hauptkörper (6) eine zweite Seite (7) umfasst, die imstande ist, der Haut eines Benutzers zugewandt zu sein, wobei das Pflaster (5) eine erste Klebeschicht (8) umfasst, die die zweite Seite (7) mindestens teilweise bedeckt, und ein abziehbares Element (9), das die Klebeschicht (8) bedeckt,
**dadurch gekennzeichnet, dass**:
- das abziehbare Element (9) mindestens zwei getrennte Teile (10) umfasst, wobei die Teile (10) einander benachbart sind,
- der Hauptkörper (6) und die erste Klebeschicht (8) einen mittigen Teil (11) umfassen, in dem ein Durchbruch (12) ausgebildet ist, der imstande ist, die Kapsel (2) aufzunehmen, wobei der Durchbruch (12) einen maximalen Durchmesser größer als 2 mm aufweist, vorzugsweise größer als 5 mm,
- das abziehbare Element (9) den Durchbruch (12) vollständig bedeckt,
- die Kapsel (2) lösbar im Durchbruch (12) aufgenommen ist, und
- das abziehbare Element (9) die Mikronadeln (4) bedeckt.

2. System (1) nach Anspruch 1, wobei sich das Pflaster (5) hauptsächlich gemäß einer Längsrichtung (13) erstreckt und wobei die beiden Teile (10) beiderseits einer Achse angeordnet sind, die parallel zur Längsrichtung (13) ist.

3. System (1) nach einem der Ansprüche 1 oder 2, wobei das Pflaster eine längliche Form gemäß der Längsrichtung (13) aufweist.

4. System (1) nach einem der Ansprüche 1 bis 3, wobei jeder Teil (10) des abziehbaren Elements (9) eine Zunge (14) umfasst, die zum Ergreifen durch einen Benutzer geeignet ist.

5. System (1) nach Anspruch 4, wobei mindestens eine der Zungen (14) mit einem Ende (15) des abziehbaren Elements (9) in Bezug auf die Längsrichtung (13) verbunden ist.

6. System (1) nach einem der Ansprüche 4 oder 5, wobei die Zunge (14) fest an einem Ende (15) des abziehbaren Elements (9) angebracht ist.

7. System (1) nach Anspruch 5 oder 6, wobei sich mindestens eine Zunge (14) hauptsächlich gemäß einer Hauptachse (16) erstreckt, die eine Komponente ungleich null gemäß der Längsrichtung (13) aufweist.

8. System (1) nach Anspruch 7, wobei der absolute Wert eines Winkels zwischen der Längsrichtung (13) und der Hauptachse (16) vorzugweise zwischen 30° und 60° liegt.

9. System (1) nach einem der Ansprüche 1 bis 8, wobei der Hauptkörper (6) eine Dicke aufweist, die größer ist als eine Dicke der Klebeschicht (8), und vorzugsweise eine Dicke über 50 µm, insbesondere über 100 µm.

10. System (1) nach einem der Ansprüche 1 bis 9, wobei das Pflaster (5) eine Kontur (17) aufweist und wobei die Teile der Kontur (17), die beiderseits des Durchbruchs (12) gemäß einer Richtung quer zur Längsrichtung (13) eingerichtet sind, Sägezähne bilden.

11. System (1) nach einem der Ansprüche 1 bis 10, wobei das abziehbare Element (9) mindestens eine äußere Schicht (18) aufweist, die gegenüber dem Hauptkörper (6) in Bezug auf den Rest des abziehbaren Elements (9) angeordnet ist, wobei die äußere Schicht (18) die Mikronadeln (4) bedeckt, wobei das abziehbare Element ebenfalls mindestens einen Abstandshalter (19) umfasst, der mindestens teilweise um den Durchbruch (12) herum eingerichtet ist, wobei eine Dicke des Abstandshalters ausgelegt ist, um einen Teil der äußeren Schicht (18), die den Durchbruch (12) bedeckt, in Bezug auf den Rest der äußeren Schicht (18) derart anzuheben, dass ein Raum zwischen der äußeren Schicht (18) und einer Spitze jeder Mikronadel (4) gebildet wird.

12. System (1) nach Anspruch 11, umfassend zwei Abstandshalter (19), die beiderseits des Durchbruchs (12) gemäß einer Richtung quer zur Längsrichtung (13) eingerichtet sind, wobei sich jeder Abstandshalter hauptsächlich gemäß der Längsrichtung (13) erstreckt.

13. Vorrichtung (20), die zur Befestigung an einer Gliedmaße bestimmt ist, umfassend:
- ein System (1) nach einem der Ansprüche 1 bis 12,
- ein Gehäuse (21), das imstande ist, lösbar mit der Kapsel (2) befestigt zu sein, in dem sich eine Batterie und ein Prozessor befinden, wobei der Prozessor ausgelegt ist, um Daten zu verarbeiten, die mit Hilfe der Messung erhalten werden, die durch eine der Mikronadeln (4) durchgeführt wurden,
- ein Band (22), das ausgelegt ist, um das Gehäuse (21) auf der Gliedmaße an Ort und Stelle zu halten.

## Claims

1. A body monitoring system (1), comprising:
- a capsule (2) for measuring a body analyte, the capsule (2) comprising a first face (3) capable of being brought into contact with the skin of a user, and microneedles (4) fixedly mounted on the first face (3),
- a patch (5) comprising a main body (6), the main body (6) comprising a second face (7) capable of being against the skin of a user, the patch (5) comprising a first layer of adhesive (8) covering at least partly the second face (7), and a peelable cover (9) covering the layer of adhesive (8),
**characterized in that**:
- the peelable cover (9) comprises at least two separate parts (10), the parts (10) being adjacent to each other,
- the main body (6) and the first layer of adhesive (8) comprise a central part (11) in which an opening (12) capable of receiving the capsule (2) is formed, the opening (12) having a maximum diameter greater than 2 mm, preferably greater than 5 mm,
- the peelable cover (9) entirely covers the opening (12),
- the capsule (2) is removably received in the opening (12), and
- the peelable cover (9) covers the microneedles (4).

2. The system (1) according to claim 1, wherein the patch (5) extends mainly along a longitudinal direction (13), and wherein the two parts (10) are arranged on either side of an axis parallel to the longitudinal direction (13).

3. The system (1) according to any of claims 1 or 2, wherein the patch has an oblong shape along the longitudinal direction (13).

4. The system (1) according to any of claims 1 to 3, wherein each part (10) of the peelable cover (9) comprises a tab (14) adapted to be gripped by a user.

5. The system (1) according to claim 4, wherein at least one of the tabs (14) is linked to one end (15) of the peelable cover (9) relative to the longitudinal direction (13).

6. The system (1) according to any of claims 4 or 5, wherein the tab (14) is fixedly mounted at one end (15) of the peelable cover (9).

7. The system (1) according to claim 5 or 6, wherein at least one tab (14) mainly extends along a main axis (16) having a non-zero component along the longitudinal direction (13).

8. The system (1) according to claim 7, wherein the absolute value of an angle formed between the longitudinal direction (13) and the main axis (16) is preferably comprised between 30° and 60°.

9. The system (1) according to any of claims 1 to 8, wherein the main body (6) has a greater thickness than a thickness of the layer of adhesive (8), and preferably a thickness greater than 50 µm, in particular greater than 100 µm.

10. The system (1) according to any of claims 1 to 9, wherein the patch (5) has a contour (17), and wherein parts of the contour (17) arranged on either side of the opening (12) along a direction transverse to the longitudinal direction (13) form sawteeth.

11. The system (1) according to any of claims 1 to 10, wherein the peelable cover (9) comprises at least one external layer (18) disposed opposite the main body (6) relative to the rest of the peelable cover (9), the external layer (18) covering the microneedles (4), the peelable cover also comprising at least one spacer (19) arranged at least partly around the opening (12), a thickness of the spacer being configured to raise a part of the external layer (18) covering the opening (12) relative to the rest of the external layer (18) so as to form a space between the external layer (18) and an top of each microneedle (4).

12. The system (1) according to claim 11, comprising two spacers (19) arranged on either side of the opening (12) along a direction transverse to the longitudinal direction (13), each spacer extending mainly along the longitudinal direction (13).

13. A device (20) intended to be attached to a limb, comprising:
- a system (1) in accordance with any of claims 1 to 12,
- a casing (21) able be removably fixed with the capsule (2), inside which there is a battery and a processor, the processor being configured to process data obtained using the measurement made by at least one of the microneedles (4),
- a strap (22) configured to hold the casing (21) in place on the limb.
